# EUROPEAN PATENT APPLICATION

(11) **EP 4 772 230 A1**
(43) Date of publication of application: **08.07.2026**
(21) Application number: 24868140.5
(22) Date of filing: 06.09.2024
(51) Int. Cl.: A61M 25/06, A61B 5/153

(54) **SHAPE HOLDING DEVICE**

(30) Priority: 19.09.2023 JP 2023150973
(71) Applicant: Terumo Kabushiki Kaisha, Tokyo 151-0072 (JP)
(72) Inventor: MIZUNO, Shinichi, Nakakoma-gun, Yamanashi 409-3853 (JP)
(74) Representative: TBK
(86) International application number: PCT/JP2024/031977
(87) International publication number: WO 2025/063062

(57) **Abstract**

The present disclosure is a shape retaining device (10) that prevents blockage of a distal end portion of a catheter body (14). The shape retaining device (10) includes: a tube (40) in which at least a distal end region including an inlet (42) is harder than a distal end portion of a catheter body (14); and a support member (50) that is fittable to a catheter hub (16), includes a fitting portion (56) communicating with the catheter hub (16) in a state of being fitted to the catheter hub (16), and supports the tube (40) so as to be displaceable with respect to the fitting portion (56).

## Description

### Technical Field

The present invention relates to a shape retaining device that retains a distal end shape of a catheter indwelled in a blood vessel.

### Background Art

A catheter assembly disclosed in JP 3958956 B2 includes a catheter body having an open distal end, a catheter hub connected to a proximal end of the catheter body, and an inner needle disposed inside the catheter.

### Citation List

### Patent Literature

Patent Literature 1: JP 3958956 B2

### Summary of Invention

### Technical Problem

In the catheter assembly of JP 3958956 B2, after the distal end of the catheter body is inserted into the blood vessel and the inner needle is removed, a suction instrument may be attached to the catheter hub, and blood may be sucked by the suction instrument. At this time, negative pressure is generated in the catheter hub and the catheter body by suction of the suction instrument, and the distal end of the catheter body may be elastically deformed by the negative pressure and squashed and blocked. Since the distal end of the catheter is blocked, there is a problem that blood cannot be sucked, and whether or not the distal end of the catheter is inserted into the blood vessel cannot be accurately determined.

An object of the present invention is to solve the above-described problem.

(1) An aspect of the present invention is a shape retaining device that is used by being connected to a catheter member including a catheter body indwelled in a blood vessel and a catheter hub fixed to a proximal end portion of the catheter body, and prevents blockage of a distal end portion of the catheter body, the shape retaining device including: a tube in which an inlet through which blood inflows, an outlet through which the blood flows out, and a lumen communicating between the inlet and the outlet are formed, and at least a distal end region including the inlet is harder than the distal end portion of the catheter body; and a support member that is fittable to the catheter hub, includes a fitting portion communicating with the catheter hub in a state of being fitted to the catheter hub, and supports the tube so as to be displaceable with respect to the fitting portion, wherein in a state where the fitting portion is fitted to the catheter hub, the tube is displaced in a direction communicating with the catheter hub with respect to the fitting portion to enter an insertion state of being inserted into the catheter body, and in the insertion state, the inlet is disposed at the same position as a distal end opening of the catheter body, at a position on a distal end side of the distal end opening, or at an internal position of the distal end portion, and the outlet is disposed on a proximal end side of the inlet.

With this shape retaining device, it is possible to prevent blockage of the distal end of the catheter with the tube at the time of suction by the suction instrument. As a result, it is possible to easily determine whether or not the distal end of the catheter has been inserted into the blood vessel by suctioning the blood using the suction instrument.

(2) The shape retaining device according to (1), wherein the outlet may be formed in a side wall of the tube, and may be disposed on the support member or the catheter hub in the insertion state.

In this way, the blood flowing through the lumen of the tube and the blood flowing between the tube and the catheter body can merge.

(3) The shape retaining device according to (1) or (2), wherein an operation portion for pushing the tube may be provided at a proximal end of the tube.

In this way, it is easy to displace the tube in the direction communicating with the catheter hub with respect to the fitting portion.

(4) The shape retaining device according to any of (1) to (3), wherein the support member may include a tube movement restriction portion that restricts movement of the tube, and in a state where the tube movement restriction portion restricts the movement of the tube, the tube may be in the insertion state.

In this way, the tube can be set at an appropriate position regardless of the operation skill of operating the tube.

(5) The shape retaining device according to any of (1) to (4), wherein the support member may include a coupling adapter in which a through-hole is formed, in the coupling adapter, the fitting portion may be provided on one end side of the through-hole, in the coupling adapter, a connection portion for connecting a suction instrument may be provided on an other end side of the through-hole, and in the coupling adapter, a tube introduction portion for introducing the tube toward the through-hole may be provided between the one end and the other end of the through-hole.

In this way, the tube can be inserted into the catheter body using the existing catheter member and suction instrument.

(6) The shape retaining device according to (5), wherein an operation portion for pushing the tube may be provided at a proximal end of the tube, a cover compressible in a direction of pushing the operation portion may be connected to the operation portion and the tube introduction portion, and the cover may cover a portion of the tube located between the operation portion and the tube introduction portion.

In this way, the tube is not exposed to the outside, which is hygienic.

(7) The shape retaining device according to (6), wherein a fixing portion for fixing the operation portion may be formed in the tube introduction portion, and in a state where the operation portion is fixed to the fixing portion, the tube may enter the insertion state.

In this way, it is possible to suppress the deviation of the relative position between the catheter body and the tube inserted into the catheter body.

(8) The shape retaining device according to any of (1) to (4), wherein the support member may include a syringe barrel including the fitting portion formed at a distal end, and a plunger movable in the syringe barrel, and the tube may penetrate the plunger.

In this way, the tube can be inserted into the catheter body without the coupling adapter. As a result, the number of components can be reduced, and downsizing can be achieved.

(9) The shape retaining device according to (8), wherein the support member may include a gasket that seals between the plunger and the syringe barrel, and a seal member that seals between the plunger and the tube.

In this way, it is possible to suppress the blood sucked into the syringe barrel from leaking to the outside of the syringe barrel.

(10) The shape retaining device according to (9), wherein the gasket and the seal member may be integrally formed.

In this way, the number of components can be reduced.

(11) The shape retaining device according to any of (8) to (10), wherein the syringe barrel may be provided with a plunger movement restriction portion that restricts movement of the plunger in a direction toward a proximal end of the syringe barrel, and in a state where the plunger movement restriction portion restricts the movement of the plunger in a direction toward the proximal end of the syringe barrel, the inlet may be disposed at the same position as the distal end opening, at a position on a distal end side of the distal end opening, or at an internal position of the distal end portion, and the outlet may be located in the syringe barrel.

In this way, the tube can be set at an appropriate position regardless of the operation skill of operating the tube.

According to an aspect of the present invention, it is possible to prevent blockage of the distal end of the catheter with the tube at the time of suction by the suction instrument. As a result, it is possible to easily determine whether or not the distal end of the catheter has been inserted into the blood vessel by suctioning the blood using the suction instrument.

### Brief Description of Drawings

[Fig. 1] Fig. 1 is a view illustrating a shape retaining device and a catheter member according to a first embodiment.
[Fig. 2] Fig. 2 is a perspective view illustrating a proximal end portion of a tube.
[Fig. 3] Fig. 3 is a view illustrating a state in which the catheter member and a suction instrument are coupled by a coupling adapter.
[Fig. 4] Fig. 4 is a view illustrating a state in which a tube introduced from the coupling adapter is inserted into the catheter member.
[Fig. 5] Fig. 5 is a view illustrating a case where blood is sucked in the state of Fig. 4.
[Fig. 6] Fig. 6 is a view illustrating a shape retaining device and a catheter member according to a second embodiment.
[Fig. 7] Fig. 7 is a view illustrating a state in which the catheter member and a suction instrument are coupled.
[Fig. 8] Fig. 8 is a view illustrating a state in which a tube is inserted into the catheter member.
[Fig. 9] Fig. 9 is a view illustrating a case where blood is sucked in the state of Fig. 8.

### Description of Embodiments

### [First Embodiment]

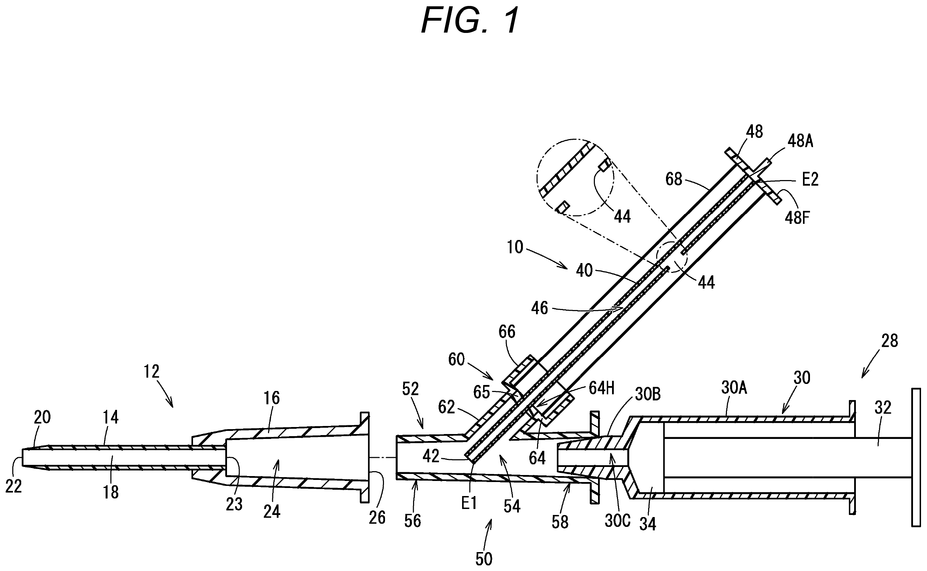

As illustrated in Fig. 1, a shape retaining device 10 according to the first embodiment is used by being connected to a catheter member 12.

The catheter member 12 includes a catheter body 14 and a catheter hub 16 connected to a proximal end of the catheter body 14. The catheter body 14 is formed in a tubular shape having a lumen 18 therein. The catheter body 14 has flexibility. The distal end of the catheter body 14 has a distal outer surface 20 tapered in a distal end direction on an outer peripheral surface. The distal end of the catheter body 14 has a distal end opening 22 opened in the distal end direction. The distal end opening 22 is disposed radially inward of the distal outer surface 20 and communicates with the lumen 18. The distal end of the catheter body 14 having the distal end opening 22 gradually becomes thinner in a radial direction in the distal end direction.

The catheter hub 16 is formed in a cylindrical shape having a hub lumen 24. The catheter hub 16 includes a proximal end opening 26 opened in a proximal end direction. The hub lumen 24 of the catheter hub 16 communicates with the lumen 18 of the catheter body 14 via a proximal end opening 23 of the catheter body 14.

The catheter member 12 is used for suction by a suction instrument 28. The suction instrument 28 is, for example, a syringe, but is not limited thereto. When the suction instrument 28 is a syringe, the suction instrument 28 includes a syringe barrel 30, a plunger 32, and a gasket 34.

The syringe barrel 30 includes a barrel body 30A and a nozzle 30B protruding from the barrel body 30A. The outer diameter of the nozzle 30B of the syringe barrel 30 is smaller than the outer diameter of the barrel body 30A. A passage 30C communicating with the inside of the barrel body 30A is formed in the nozzle 30B of the syringe barrel 30. The plunger 32 is provided to be movable inside the barrel body 30A. The gasket 34 seals between the syringe barrel 30 and the plunger 32. The gasket 34 is fixed to the distal end of the plunger 32.

The shape retaining device 10 is a device that prevents blockage of a distal end portion of the catheter body 14. The shape retaining device 10 includes a tube 40 and a support member 50.

The tube 40 is a member to be inserted into the lumen 18 of the catheter body 14. The tube 40 is harder than at least the distal end portion of the catheter body 14. An inlet 42 through which blood flows is formed in the tube 40. The inlet 42 is located at a distal end E1 of the tube 40. An outlet 44 through which blood flows out is formed in the tube 40. The outlet 44 is formed in a side wall of the tube 40. A lumen 46 is formed in the tube 40. The lumen 46 of the tube 40 communicates the inlet 42 and the outlet 44. Note that the lumen 46 of the tube 40 is formed from the distal end E1 to a proximal end E2 of the tube 40, but is not limited thereto. The lumen 46 of the tube 40 is formed from the distal end E1 to the outlet 44 of the tube 40, and may not be formed from the outlet 44 to the proximal end E2.

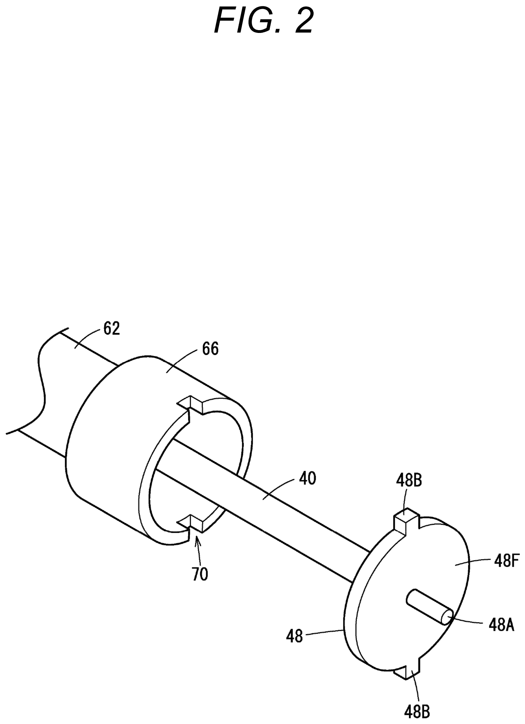

As illustrated in Fig. 2, an operation portion 48 for pushing the tube 40 is provided at the proximal end E2 of the tube 40. The operation portion 48 is formed in, for example, a plate shape. The operation portion 48 has a pressure receiving surface 48F that receives the operation of pushing the tube 40. The pressure receiving surface 48F is located on the side opposite to the proximal end E2 of the tube 40 in the operation portion 48. The operation portion 48 may be provided with a grip portion 48A. The grip portion 48A is formed in, for example, a rod shape extending from the pressure receiving surface 48F in a direction opposite to a direction in which the tube 40 is pushed. In order to restrict the movement of the tube 40, the operation portion 48 may be provided with a pair of protrusions 48B. For example, the pair of protrusions 48B protrudes from the operation portion 48 in a direction intersecting the direction in which the tube 40 is pushed.

As illustrated in Fig. 1, the support member 50 includes a coupling adapter 52. The coupling adapter 52 is an adapter capable of coupling the catheter hub 16 and the suction instrument 28. The coupling adapter 52 is formed in a tubular shape. A through-hole 54 is formed in the coupling adapter 52. The through-hole 54 extends in a direction along the extending direction of the coupling adapter 52. The coupling adapter 52 is provided with a fitting portion 56, a connection portion 58, and a tube introduction portion 60.

The fitting portion 56 is a portion formed so as to be fittable to the catheter hub 16. The fitting portion 56 is provided in a portion located on one end side of the through-hole 54 in the coupling adapter 52. The fitting portion 56 is formed to be insertable into the proximal end opening 26 of the catheter hub 16. When the fitting portion 56 is inserted into the proximal end opening 26 of the catheter hub 16, the fitting portion is liquid-tightly fitted to an inner peripheral surface of the catheter hub 16. In this case, the hub lumen 24 of the catheter hub 16 communicates with the through-hole 54 of the coupling adapter 52. In addition, a gap between the catheter hub 16 and the fitting portion 56 is sealed in a liquid-tight manner.

The connection portion 58 is a portion formed to be connectable with the suction instrument 28. The connection portion 58 is provided in a portion of the coupling adapter 52 located on the other end side of the through-hole 54. When the suction instrument 28 is a syringe, the connection portion 58 is connected to the nozzle 30B of the syringe barrel 30. When the suction instrument 28 is connected to the connection portion 58, suction by the suction instrument 28 can be performed from the catheter body 14 through the through-hole 54.

The tube introduction portion 60 is a portion formed so that the tube 40 can be introduced toward the through-hole 54. The tube introduction portion 60 is provided in a portion of the coupling adapter 52 located between one end and the other end of the through-hole 54. The tube introduction portion 60 includes an introduction tube 62, a support base portion 64, and a tube movement restriction portion 66.

One end of the introduction tube 62 is connected to an outer peripheral surface of the coupling adapter 52. The other end of the introduction tube 62 is connected to the support base portion 64. A tube insertion hole 64H is formed in the support base portion 64. The tube insertion hole 64H communicates with the through-hole 54 through the inside of the introduction tube 62. The tube 40 is inserted into the introduction tube 62 and the tube insertion hole 64H. The proximal end E2 of the tube 40 is disposed outside. When the operation portion 48 provided at the proximal end E2 of the tube 40 is pushed, the tube 40 is introduced into the through-hole 54. The tube insertion hole 64H is provided with a valve body 65 that seals between the tube 40 and the support base portion 64. Note that the valve body 65 may not be provided.

The introduction tube 62 extends in a direction inclined with respect to the extending direction of the through-hole 54. The introduction tube 62 is inclined so as to be away from the coupling adapter 52 toward the other end of the through-hole 54. As a result, the distal end E1 of the tube 40 introduced into the through-hole 54 is easily displaced toward the fitting portion 56.

A cover 68 that can be compressed in a direction of pushing the operation portion 48 is connected between the operation portion 48 provided at the proximal end E2 of the tube 40 and the support base portion 64. The cover 68 can be formed using a material such as polyethylene or polypropylene, for example, into a tubular film or the like having flexibility. The cover 68 covers a portion of the tube 40 located between the operation portion 48 and the support base portion 64. Therefore, the tube 40 is not exposed to the outside and is hygienic.

The tube movement restriction portion 66 is a portion that restricts the movement of the tube 40. The tube movement restriction portion 66 extends from the support base portion 64 toward the operation portion 48. The tube movement restriction portion 66 may be formed integrally with the support base portion 64. As illustrated in Fig. 2, the tube movement restriction portion 66 is formed in a tubular shape, for example. The tube movement restriction portion 66 is formed with fixing portions 70 for fixing the operation portion 48. For example, as illustrated in Fig. 2, when the operation portion 48 is provided with the pair of protrusions 48B, the fixing portions 70 are formed as recessed portions to be fitted with the pair of protrusions 48B at the proximal end of the tube movement restriction portion 66.

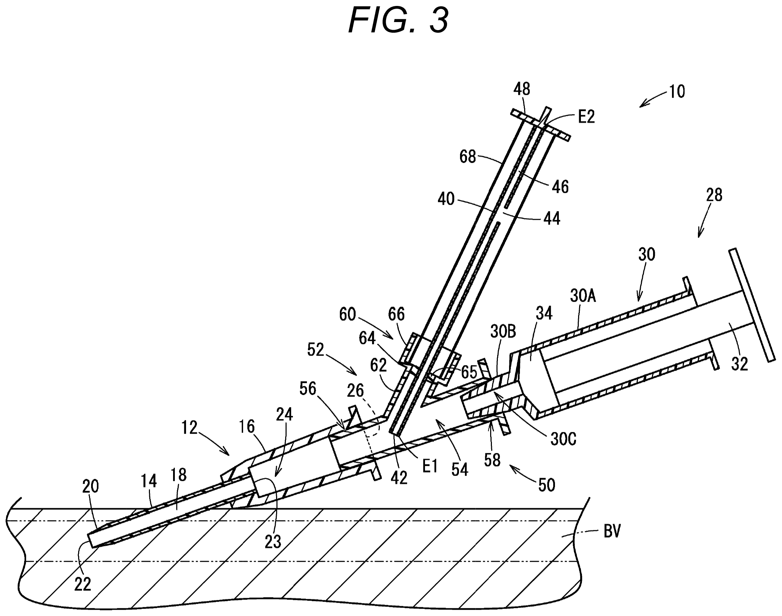

Next, use of the shape retaining device 10 will be described. First, the user punctures a blood vessel BV of a living body with the catheter assembly in which a needle member (not illustrated) is inserted into the catheter member 12, and then removes the needle member from the inside of the catheter member 12. As a result, as illustrated in Fig. 3, the catheter member 12 is indwelled in a state where the distal end of the catheter body 14 is disposed in the blood vessel BV of the living body.

Next, the user couples the catheter member 12 and the suction instrument 28 via the coupling adapter 52. In this case, the fitting portion 56 of the coupling adapter 52 is inserted into the catheter hub 16. In addition, the suction instrument 28 is connected to the connection portion 58 of the coupling adapter 52. At this stage, the tube 40 is in an initial state. In the initial state, the inlet 42 of the tube 40 is located in the through-hole 54 of the coupling adapter 52, and the outlet 44 of the tube 40 is located in the cover 68. Note that the inlet 42 of the tube 40 in the initial state may be located in the tube introduction portion 60 such as the introduction tube 62.

Next, the user pushes the operation portion 48 to start insertion of the tube 40 into the catheter body 14. When the operation portion 48 starts to be pushed, the distance of the operation portion 48 with respect to the tube movement restriction portion 66 of the tube introduction portion 60 gradually decreases. According to this decrease, the cover 68 disposed between the operation portion 48 and the tube movement restriction portion 66 is gradually compressed. In addition, the tube 40 moves according to the decrease in distance of the operation portion 48 with respect to the tube movement restriction portion 66. In this case, the tube 40 is guided by the inner peripheral surface of the coupling adapter 52 surrounding the through-hole 54 and advances through the through-hole 54 toward the proximal end opening 26 of the catheter hub 16. That is, the tube 40 is displaced in the direction (distal end direction) communicating with the catheter hub 16 with respect to the fitting portion 56 of the coupling adapter 52.

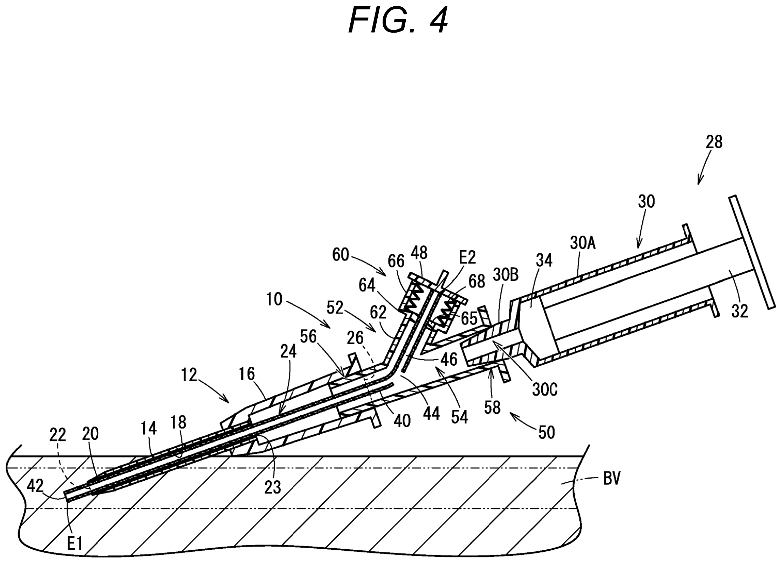

As illustrated in Fig. 4, when the operation portion 48 comes into contact with the tube movement restriction portion 66 of the tube introduction portion 60, the movement of the tube 40 is restricted, and the insertion of the tube 40 into the catheter body 14 is ended. In this case, the tube 40 is in an insertion state of being inserted into the catheter body 14. That is, when the movement of the tube 40 is restricted by the tube movement restriction portion 66, the tube 40 enters the insertion state. Therefore, the tube 40 can be set at an appropriate position regardless of the operation skill of operating the operation portion 48.

In the insertion state of the tube 40, the inlet 42 of the tube 40 is located on the distal end side of the distal end opening 22 of the catheter body 14. The outlet 44 of the tube 40 is located in the through-hole 54 of the coupling adapter 52 on the proximal end side of the proximal end opening 23 of the catheter body 14. Note that the position of the inlet 42 of the tube 40 in the insertion state of the tube 40 may be the same position as the distal end opening 22 of the catheter body 14. In addition, the position of the outlet 44 of the tube 40 in the insertion state of the tube 40 may be the hub lumen 24 of the catheter hub 16.

The operation portion 48 in contact with the tube movement restriction portion 66 is fixed to the fixing portions 70 formed in the tube movement restriction portion 66. As illustrated in Fig. 2, in the present embodiment, the operation portion 48 is fixed by fitting the pair of protrusions 48B provided on the operation portion 48 into the recessed portions (fixing portions 70) formed at the proximal end of the tube movement restriction portion 66. Therefore, displacement of the relative position between the catheter body 14 and the tube 40 inserted into the catheter body 14 is suppressed.

When the tube 40 enters the insertion state, the user starts suction using the suction instrument 28. In the present embodiment, the tube 40 harder than the catheter body 14 is in the insertion state in which the tube 40 is inserted into the catheter body 14. Therefore, it is possible to suppress the distal end of the catheter body 14 from being blocked due to the negative pressure generated in response to the suction by the suction instrument 28. As a result, it is possible to determine whether or not the distal end of the catheter body 14 is inserted into the blood vessel according to the presence or absence of inflow of blood into the lumen 46 of the tube 40 or the amount of inflow blood.

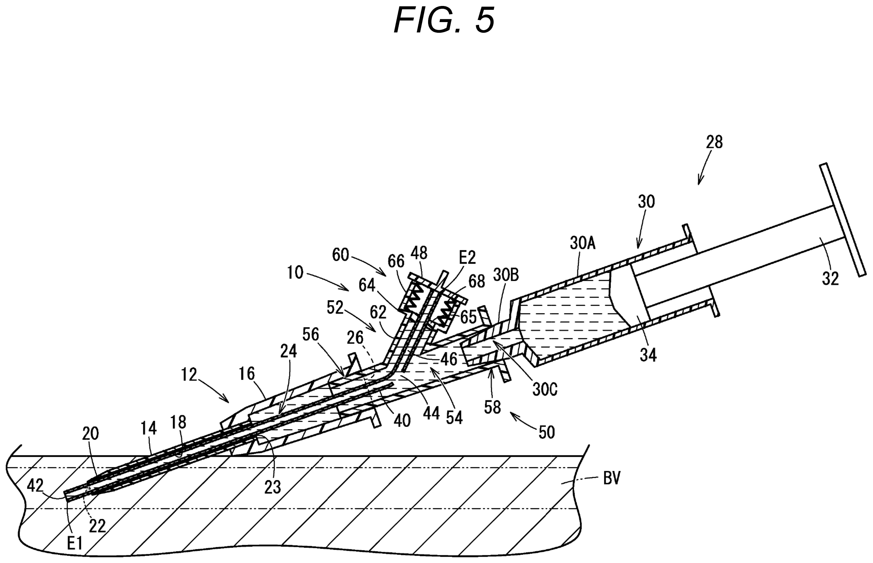

As illustrated in Fig. 5, when blood flows into the lumen 46 of the tube 40, the blood flows out from the outlet 44 to the through-hole 54 of the coupling adapter 52. In addition, when blood flows between the tube 40 and the catheter body 14, the blood flows out to the through-hole 54 via the hub lumen 24. That is, the blood flowing through the lumen 46 of the tube 40 and the blood flowing between the tube 40 and the catheter body 14 merge at the through-hole 54 of the coupling adapter 52. The blood flowing through the through-hole 54 is sucked by the suction instrument 28. When the suction instrument 28 is a syringe, the blood flowing through the through-hole 54 is stored inside the syringe barrel 30 through the nozzle 30B of the syringe barrel 30. Note that, in the insertion state of the tube 40, the outlet 44 is desirably close to the distal end of the suction instrument 28. When the outlet 44 is close to the distal end of the suction instrument 28, the blood can be efficiently sucked.

### [Second Embodiment]

Next, the shape retaining device 10 according to a second embodiment will be described. In the present embodiment, constituent elements equivalent to those in the first embodiment are denoted by the same reference numerals as those used in the first embodiment. In addition, in the present embodiment, description overlapping with the first embodiment will be omitted.

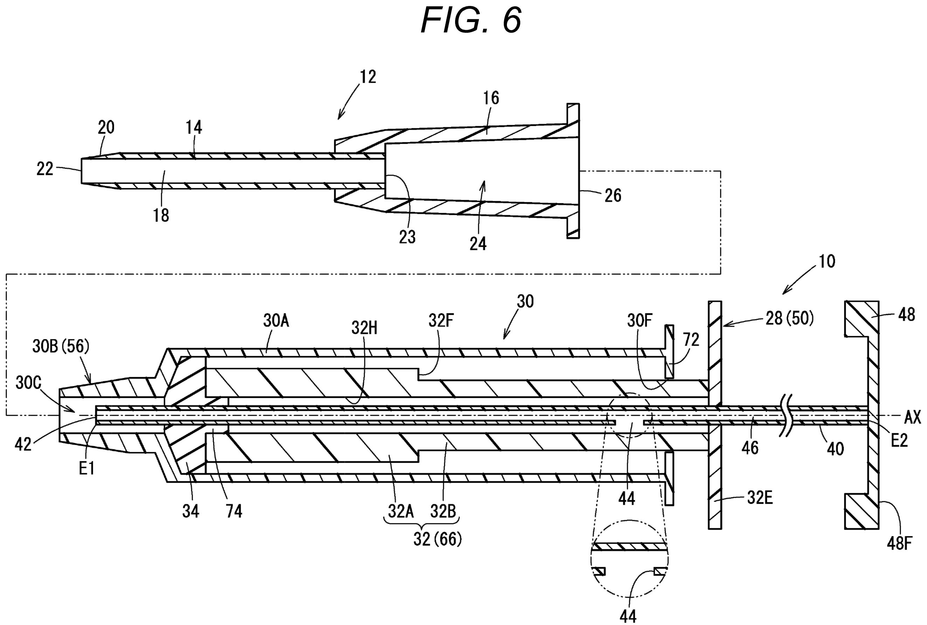

As illustrated in Fig. 6, in the present embodiment, the support member 50 does not include the coupling adapter 52 of the first embodiment. The tube 40 is supported by the suction instrument 28. That is, in the present embodiment, the support member 50 is the suction instrument 28.

The suction instrument 28 of the present embodiment is a syringe, and includes the syringe barrel 30, the plunger 32, and the gasket 34 as in the first embodiment. A tube insertion hole 32H is formed in the plunger 32 and the gasket 34 along an axis AX of the syringe. The tube 40 is inserted into the tube insertion hole 32H and penetrates the plunger 32 and the gasket 34. The tube 40 is disposed along the axis AX of the syringe and is movable relative to the plunger 32.

In the initial state of the tube 40, the inlet 42 and the outlet 44 of the tube 40 are located inside the syringe barrel 30 and do not protrude to the outside of the syringe barrel 30. Therefore, a distal end region of the tube 40 inserted into the catheter body 14 is not exposed to the outside, which is hygienic.

In the suction instrument 28 of the present embodiment, the nozzle 30B of the syringe barrel 30 can be inserted into the proximal end opening 26 of the catheter hub 16. When the nozzle 30B of the syringe barrel 30 is inserted into the proximal end opening 26 of the catheter hub 16, the nozzle 30B is liquid-tightly fitted to the inner peripheral surface of the catheter hub 16. In this case, the hub lumen 24 of the catheter hub 16 communicates with the inside of the syringe barrel 30. In addition, a gap between the catheter hub 16 and the nozzle 30B of the syringe barrel 30 is sealed in a liquid-tight manner. That is, the nozzle 30B of the syringe barrel 30 corresponds to the fitting portion 56 of the first embodiment.

The suction instrument 28 of the present embodiment newly includes a plunger movement restriction portion 72 and a seal member 74.

The plunger movement restriction portion 72 restricts the movement of the plunger 32 in a direction toward the proximal end of the syringe barrel 30. The plunger movement restriction portion 72 is provided in the barrel body 30A. The plunger movement restriction portion 72 extends from an inner wall on the proximal end side of the barrel body 30A to a proximal end opening 30F of the syringe barrel 30. Therefore, the proximal end opening 30F of the syringe barrel 30 is smaller than a case where the plunger movement restriction portion 72 is not provided. Note that the plunger movement restriction portion 72 may be formed integrally with the barrel body 30A or may be formed separately. In addition, the shape of the plunger movement restriction portion 72 may be annular or convex, and is not particularly limited.

The plunger 32 has different outer diameters at a distal end portion 32A and a proximal end portion 32B. The outer diameter of the distal end portion 32A of the plunger 32 is larger than the outer diameter of the proximal end portion 32B of the plunger 32. The proximal end portion 32B of the plunger 32 can be inserted through the proximal end opening 30F of the syringe barrel 30. On the other hand, the distal end portion 32A of the plunger 32 cannot be inserted through the proximal end opening 30F of the syringe barrel 30.

In accordance with the movement of the plunger 32 in the direction toward the proximal end of the syringe barrel 30, when a surface 32F of the distal end portion 32A facing a proximal end 32E of the plunger 32 abuts on the plunger movement restriction portion 72, the movement of the plunger 32 is restricted.

The seal member 74 liquid-tightly seals between the plunger 32 and the tube 40. The gasket 34 and the seal member 74 may be formed integrally or may be formed separately. When the gasket 34 and the seal member 74 are integrally formed, the number of parts can be reduced.

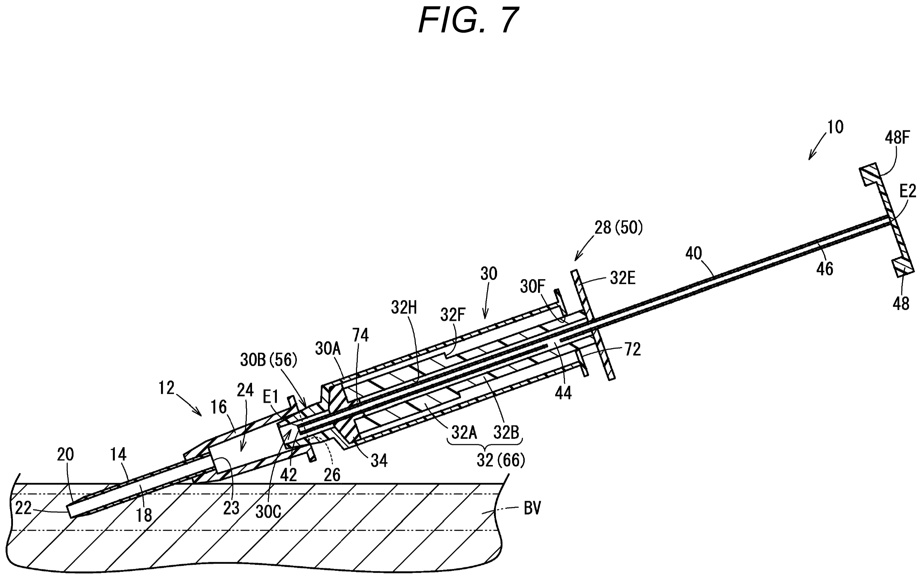

Next, use of the shape retaining device 10 will be described. First, the user punctures a blood vessel BV of a living body with the catheter assembly in which a needle member (not illustrated) is inserted into the catheter member 12, and then removes the needle member from the inside of the catheter member 12. As a result, as illustrated in Fig. 7, first, the catheter member 12 is indwelled in a state where the distal end of the catheter body 14 is disposed in the blood vessel BV of the living body.

The user then couples the suction instrument 28 to the catheter hub 16. In this case, the nozzle 30B of the syringe barrel 30 is inserted into the proximal end opening 26 of the catheter hub 16, and the nozzle 30B of the syringe barrel 30 is fitted to the catheter hub 16. At this stage, the tube 40 is in the initial state. In the initial state, the inlet 42 and the outlet 44 of the tube 40 are located inside the syringe barrel 30.

Next, the user pushes the operation portion 48 to start insertion of the tube 40 into the catheter body 14. When the operation portion 48 starts to be pushed in the distal end direction, the tube 40 moves. In this case, the tube 40 advances toward the proximal end opening 26 of the catheter hub 16 along the axis AX of the syringe. That is, the tube 40 is displaced in a direction communicating with the catheter hub 16 with respect to the syringe barrel 30. Note that even when the operation portion 48 is pushed, the plunger 32 abuts against a wall portion on the distal end side of the syringe barrel 30 and does not advance toward the proximal end opening 26 of the catheter hub 16.

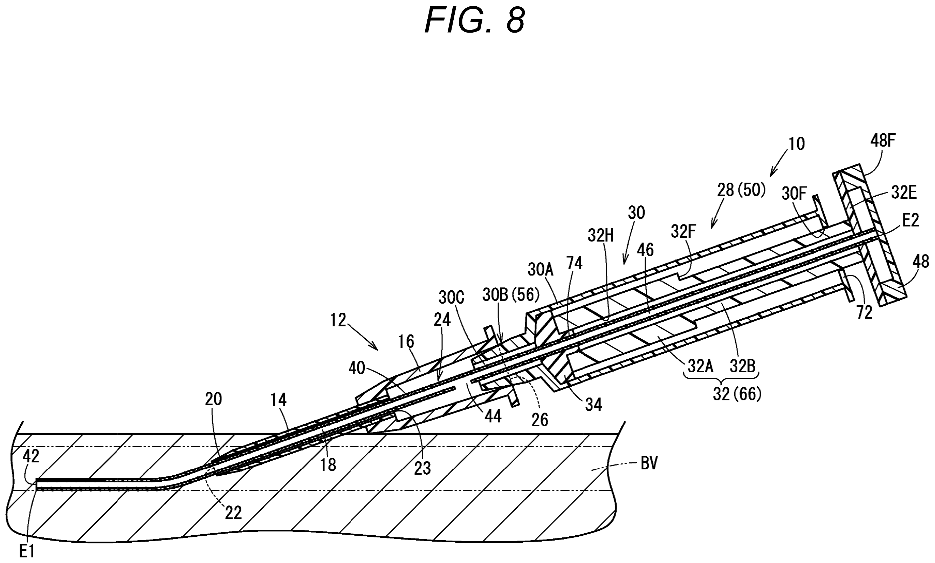

As illustrated in Fig. 8, when the operation portion 48 comes into contact with the proximal end 32E of the plunger 32, the movement of the tube 40 is restricted, and the insertion of the tube 40 into the catheter body 14 is ended. In this case, the tube 40 is in an insertion state of being inserted into the catheter body 14. That is, when the movement of the tube 40 is restricted by the plunger 32, the tube 40 enters the insertion state. The plunger 32 corresponds to the tube movement restriction portion 66 of the first embodiment. Therefore, as in the first embodiment, the tube 40 can be set at an appropriate position regardless of the operation skill of operating the operation portion 48.

In the insertion state of the tube 40, the inlet 42 of the tube 40 is located on the distal end side of the distal end opening 22 of the catheter body 14. The outlet 44 of the tube 40 is located in the hub lumen 24 of the catheter hub 16 on the proximal end side of the proximal end opening 23 of the catheter body 14.

When the tube 40 enters the insertion state, the user pulls the plunger 32 in a direction toward the proximal end of the syringe barrel 30 to start suction using the suction instrument 28. In the present embodiment, as in the first embodiment, the tube 40 harder than the catheter body 14 enters the insertion state of being inserted into the catheter body 14. Therefore, it is possible to suppress the distal end of the catheter body 14 from being blocked due to the negative pressure generated in response to the suction by the suction instrument 28. As a result, it is possible to determine whether or not the distal end of the catheter body 14 is inserted into the blood vessel according to the presence or absence of inflow of blood into the lumen 46 of the tube 40 or the amount of inflow blood.

As described above, in the insertion state of the tube 40, the operation portion 48 provided at the proximal end E2 of the tube 40 is in contact with the proximal end 32E of the plunger 32. Therefore, when the movement of the plunger 32 in the direction toward the proximal end of the syringe barrel 30 is started, the tube 40 also moves in the direction toward the proximal end of the syringe barrel 30 following the movement of the plunger 32.

When suction by the suction instrument 28 is started and blood flows into the lumen 46 of the tube 40, the blood flows out from the outlet 44. The blood flowing out of the outlet 44 merges with blood flowing between the tube 40 and the catheter body 14 from the distal end opening 22 of the catheter body 14, and the merged blood flows out to the hub lumen 24 of the catheter hub 16. The blood flowing into the hub lumen 24 of the catheter hub 16 is stored inside the syringe barrel 30 via the passage 30C of the nozzle 30B of the syringe barrel 30.

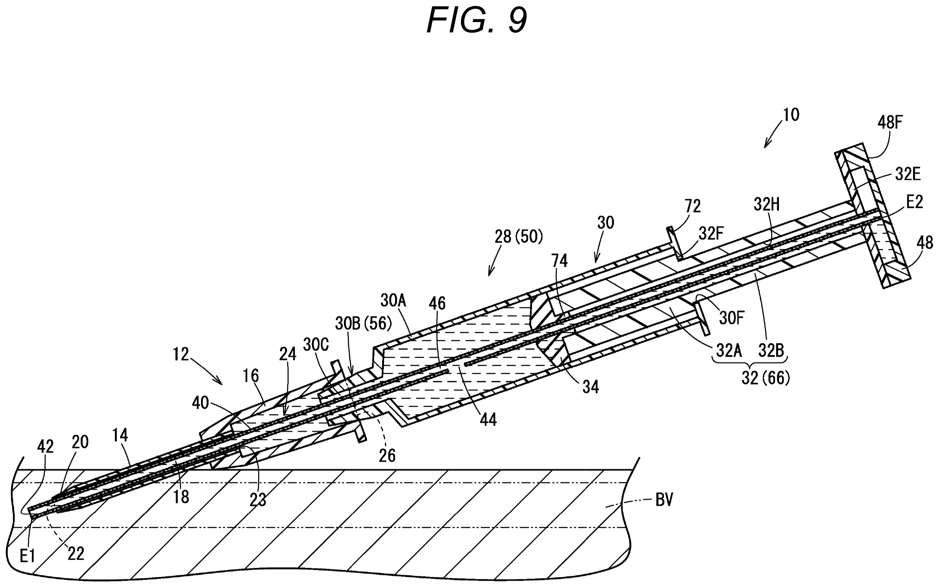

As illustrated in Fig. 9, in accordance with the movement of the plunger 32 in the direction toward the proximal end of the syringe barrel 30, when a surface 32F of the distal end portion 32A facing the proximal end 32E of the plunger 32 abuts on the plunger movement restriction portion 72, the movement of the plunger 32 is restricted. As a result, the suction by the suction instrument 28 ends. When the movement of the plunger 32 is restricted, the tube 40 following the movement of the plunger 32 also stops. Note that even when the movement of the plunger 32 is restricted, the tube 40 is movable in a direction of removal from the plunger 32.

In a state where the movement of the plunger 32 is restricted, the insertion state of the tube 40 with respect to the catheter body 14 is maintained. The inlet 42 of the tube 40 is located on the distal end side of the distal end opening 22 of the catheter body 14. The outlet 44 of the tube 40 is located in the barrel body 30A on the proximal end side of the proximal end opening 23 of the catheter body 14. Therefore, it is easy to store blood inside the syringe barrel 30. Note that, in the insertion state of the tube 40, the inlet 42 of the tube 40 may be disposed at the same position as the distal end opening 22 of the catheter body 14.

As described above, in the present embodiment, the tube 40 is supported by the suction instrument 28 so as to be insertable into the catheter body 14. Therefore, the same effects as those of the first embodiment can be obtained without the coupling adapter 52 of the first embodiment. As a result, in the present embodiment, the number of components can be reduced as compared with the first embodiment, and downsizing can be achieved.

### [Modified Embodiment]

The above embodiments may be modified as described below.

For example, only a distal end region of the tube 40 may be harder than the catheter body 14. In this case, a region other than the distal end region of the tube 40 may have the same hardness as the catheter body 14 or may be softer than the catheter body 14. Note that the distal end region of the tube 40 is equal to or larger than a region from the distal end E1 of the tube 40 to a position separated by a distance corresponding to the length in the extending direction of the catheter body 14.

In addition, the outer diameter of the tube 40 may not be the same from the distal end E1 of the tube 40 to the proximal end E2 of the tube 40. Similarly, the inner diameter of the tube 40 may not be the same from the distal end E1 of the tube 40 to the proximal end E2 of the tube 40.

In addition, in the insertion state in which the tube 40 is inserted into the catheter body 14, the inlet 42 of the tube 40 may be disposed at an internal position of the distal end portion of the catheter body 14. In this case, the inlet 42 is located within a range from the distal end of the catheter body 14 to a position separated by a distance corresponding to half of the inner diameter of the catheter body 14. Note that the position of the inlet 42 is preferably closer to the distal end opening 22 of the catheter body 14.

In the second embodiment, the fixing portions 70 may be provided at the proximal end 32E of the plunger 32. In this case, as in the first embodiment, it is possible to suppress the deviation of the relative position between the catheter body 14 and the tube 40 inserted into the catheter body 14.

Note that the present invention is not limited to the above disclosure and can take various configurations without departing from the gist of the present invention.

## Claims

1. A shape retaining device that is used by being connected to a catheter member including a catheter body indwelled in a blood vessel and a catheter hub fixed to a proximal end portion of the catheter body, and prevents blockage of a distal end portion of the catheter body, the shape retaining device comprising:
a tube in which an inlet through which blood inflows, an outlet through which the blood flows out, and a lumen communicating between the inlet and the outlet are formed, and at least a distal end region including the inlet is harder than the distal end portion of the catheter body; and
a support member that is fittable to the catheter hub, includes a fitting portion communicating with the catheter hub in a state of being fitted to the catheter hub, and supports the tube so as to be displaceable with respect to the fitting portion, wherein
in a state where the fitting portion is fitted to the catheter hub, the tube is displaced in a direction communicating with the catheter hub with respect to the fitting portion to enter an insertion state of being inserted into the catheter body, and
in the insertion state, the inlet is disposed at a same position as a distal end opening of the catheter body, at a position on a distal end side of the distal end opening, or at an internal position of the distal end portion, and the outlet is disposed on a proximal end side of the inlet.

2. The shape retaining device according to claim 1,
wherein
the outlet is formed in a side wall of the tube, and is disposed on the support member or the catheter hub in the insertion state.

3. The shape retaining device according to claim 1,
wherein
an operation portion for pushing the tube is provided at a proximal end of the tube.

4. The shape retaining device according to claim 1,
wherein
the support member includes a tube movement restriction portion that restricts movement of the tube, and
in a state where the tube movement restriction portion restricts the movement of the tube, the tube is in the insertion state.

5. The shape retaining device according to claim 2, wherein
the support member includes a coupling adapter in which a through-hole is formed,
in the coupling adapter, the fitting portion is provided on one end side of the through-hole,
in the coupling adapter, a connection portion for connecting a suction instrument is provided on an other end side of the through-hole, and
in the coupling adapter, a tube introduction portion for introducing the tube toward the through-hole is provided between the one end and the other end of the through-hole.

6. The shape retaining device according to claim 5,
wherein
an operation portion for pushing the tube is provided at a proximal end of the tube,
a cover compressible in a direction of pushing the operation portion is connected to the operation portion and the tube introduction portion, and
the cover covers a portion of the tube located between the operation portion and the tube introduction portion.

7. The shape retaining device according to claim 6,
wherein
a fixing portion for fixing the operation portion is formed in the tube introduction portion, and
in a state where the operation portion is fixed to the fixing portion, the tube enters the insertion state.

8. The shape retaining device according to claim 2,
wherein
the support member includes a syringe barrel including the fitting portion formed at a distal end, and a plunger movable in the syringe barrel, and
the tube penetrates the plunger.

9. The shape retaining device according to claim 8,
wherein
the support member includes a gasket that seals between the plunger and the syringe barrel, and a seal member that seals between the plunger and the tube.

10. The shape retaining device according to claim 9, wherein
the gasket and the seal member are integrally formed.

11. The shape retaining device according to claim 8, wherein
the syringe barrel is provided with a plunger movement restriction portion that restricts movement of the plunger in a direction toward a proximal end of the syringe barrel, and
in a state where the plunger movement restriction portion restricts the movement of the plunger in a direction toward the proximal end of the syringe barrel, the inlet is disposed at a same position as the distal end opening, at a position on a distal end side of the distal end opening, or at an internal position of the distal end portion, and the outlet is located in the syringe barrel.
